# EUROPEAN PATENT APPLICATION

(11) **EP 4 190 305 A1**
(43) Date of publication of application: **07.06.2023**
(21) Application number: 21849693.3
(22) Date of filing: 08.07.2021
(51) Int. Cl.: A61K 6/00, A61K 6/17

(54) **TOOTH-WHITENING GEL COMPRISING MODIFIED NIOBIUM COMPOUNDS, METHOD AND USE**

(30) Priority: 28.07.2020 BR 102020015367
(71) Applicant: Universidade Federal De Minas Gerais - UFMG, 31270-901 Belo Horizonte (BR); Ondonto Tech Pesquisa e Inovação, Ltda, 30140-120 Belo Horizonte, MG (BR)
(72) Inventor: HEITMANN RODRIGUES, Ana Pacheli, 31270-901 Belo Horizonte (BR); ALVES DE OLIVEIRA, Luiz Carlos, 31270-150 Belo Horizonte (BR); BELCHIOR, Jadson Claudio, 31340-300 Belo Horizonte (BR); MORGAN DOS SANTOS ALVES, Luis Fernando, 31270-901 Belo Horizonte (BR); MAUAD DE ABREU, Fernando Antonio, 31270-901 Belo Horizonte (BR)
(74) Representative: Clarke Modet & Co.
(86) International application number: PCT/IB2021/056144
(87) International publication number: WO 2022/023847

(57) **Abstract**

The present technology relates to a product applied for teeth whitening based on the generation of active oxygen in the form of oxygen radical species in situ. The whitening gel comprises modified niobium compounds and its activity is maximized mainly if it is applied with hydrogen peroxide in low concentrations, from 1 to 10% (preferably 3% by mass), with stabilization by a thickener, preferably carbopol. The whitening gel can be used in teeth whitening without the need for additional light to the treatment, which allows its use in teeth whitening with greater efficiency than commercial products and with a significant potential to reduce side effects (hypersensitivity and inflammation). The developed product did not show cytotoxicity, and no significant inflammatory effect was observed in the pulp tissue. The present technology also describes a method for producing the whitening gel.

## Description

The present technology relates to a product applied for teeth whitening based on the generation of active oxygen in the form of oxygen radical species in situ. The whitening gel comprises modified niobium compounds and its activity is maximized mainly if it is applied with hydrogen peroxide in low concentrations, from 1 to 10% (preferably 3% by mass), with stabilization by a thickener, preferably carbopol. The whitening gel can be used in teeth whitening without the need for additional light to the treatment, which allows its use in teeth whitening with greater efficiency than commercial products and with a significant potential to reduce side effects (hypersensitivity and inflammation). The developed product did not show cytotoxicity, and no significant inflammatory effect was observed in the pulp tissue. The present technology also describes a method for producing the whitening gel.

In recent years, there has been a significant increase in the demand for teeth whitening in aesthetic clinics and dental offices. However, in commercial products such as whitening or lightening gels, high concentrations (6 to 35%) of hydrogen peroxide (H₂O₂) are used in their formulations. Exposure of teeth to peroxide can cause side effects such as sensitivity, damage to the pulp, tooth enamel and periodontal tissue. In the state of the art, there are several studies highlighting the importance of minimizing these adverse effects using materials based on inorganic compounds (Application of Titanium Dioxide Nanotubes to Tooth Whitening, Nano Biomedicine, Volume 6, Issue 2, 12 December 2014, Pages 63-72 ) (Synthesis of metal ion-histidine complex functionalized mesoporous silica nanocatalysts for enhanced light free tooth bleaching, Acta Biomaterialia, Volume 7, Issue 5, 7 January 2011, Pages 2276-2284)

In the state of the art, it is also possible to find several studies highlighting the use of niobium compounds for use in oxidation reactions, including organic pollutants present in industrial effluents *(*Photocatalytic degradation of hazardous Ponceu-S dye from industrial wastewater using nanosized niobium pentoxide with carbon, Desalination, Volume 269, Issue 1-3, 4 November 2010, Pages 276-283) (Amphiphilic niobium oxyhydroxide as a hybrid catalyst for sulfur removal from fuel in a biphasic system, Applied Catalysis B: Environmental, Volume 147, 6 August 2013, Pages 43-48).

The use of reactive materials acting in teeth whitening is reported in the literature in different documents. Document US20040180008, from 2004, entitled *"Dental bleaching agent kit and the method for bleaching teeth"* and US20060222604, from 2002, with the following title *"Method for bleaching teeth and bleaching agent for teeth"* report the combined use of titanium compounds and visible light based on the photocatalysis reaction. In addition, the importance of using peroxides to ensure teeth whitening is emphasized.

The invention related to patent US5645428, from 1995, entitled "Method *for whitening teeth"* reports the efficiency of teeth whitening using a mixture of peroxides with different catalysts exposed to argon laser radiation and also with carbon dioxide laser. This method employs other materials including buffers, stabilizers, desensitizers and thickeners.

In patent US5032178, from 1990, entitled *"Dental composition system and method for bleaching teeth",* materials from the manganese sulfate and iron sulfate classes are described, which were used together with other products, including hydrogen peroxide and presence of visible radiation in teeth whitening.

Document BR102013027175-6, filed in 2013, entitled *"Teeth whitening accelerator",* relates to a mixture of ferric nitrate and cupric nitrate together with hydrogen peroxide promoting efficient whitening.

Natural catalysts such as enzymes of the peroxidases family such as catalase are described in the document BR102014010685-5, filed in 2014, entitled *"Teeth whitening maximizer",* having a rapid decomposition of hydrogen peroxide or carbamide peroxide with efficient action of teeth whitening.

Document PI0801862-6, from 2008, entitled *"Teeth whitening gel with micro or nano solid particles that absorb energy and is thermally conductive",* relates to conductive inorganic, ceramic or organic micro or nanoparticles that are incorporated into the peroxide-based whitening gel, which, in addition to providing greater efficiency in teeth whitening, minimizes side effects, such as hypersensitivity.

The use of materials based on niobium compounds, highlighting the unprecedented use of the oxalate salt (which could be niobium pentoxide, niobic acid and oxyhydroxide) in the activation of hydrogen peroxide or organic peroxides, proposed in the present technology, applied to teeth whitening is unprecedented, mainly with the generation of radical oxygen in situ due to the reaction of niobium with H₂O₂, as well as the stabilization of oxygen species with commercial carbopol. Another novelty of the present invention consists in the fact that the added hydrogen peroxide is decomposed by the reaction with niobium species, generating active oxygen species, decreasing the presence of free peroxide, causing the sensitivity to be eliminated. In this way, it is possible to reduce the side effects of hypersensitivity during treatment, in addition to allowing the production of a material with low cost, which can facilitate its commercialization.

The oxygen species generated in the dissociation of H₂O₂ have an oxidizing action capable of breaking the chemical bonds of the molecular chains of the chromophore groups (substances that give teeth color). Low concentrations of peroxide are sufficient for this effect (approximately 2% by mass) in the product presented due to three technological innovations: (i) the niobium compound acts efficiently and quickly in its activation to generate reactive radical species; (ii) decrease in free peroxide, decreasing sensitivity during bleaching (iii) carbopol stabilizes radicals preventing their decomposition, which enhances teeth whitening.

In addition, with the use of this gel in teeth whitening there is no need to use radiation for the decomposition of the peroxide, significant change in pH and with that it is possible to reduce the treatment time in the office or at home. These features provide the product generated in the present technology with great commercial potential.

The present technology relates to the development of material using sources of niobium as raw material to obtain the whitening nanoparticles. The use of niobium compounds in the production of niobium-containing teeth whitening gel, as proposed in the present technology, is not reported in the scientific literature and may promote another commercial application for this important chemical element, which is currently used mainly in the metallurgical industry.

### BRIEF DESCRIPTION OF DRAWINGS

**Figure 1** shows an applicator syringe containing teeth whitening gel.
**Figure 2** presents parameters of brightness, intensity of color/luminosity and shade obtained through the VITA 3D MASTER scale using different niobium compounds.
**Figure 3** shows photographs of bovine teeth before and after treatment with materials G1 (A) and G3 (B).
**Figure 4** shows SEM images of the teeth of rats submitted to teeth whitening with whitening gel G1 (A), commercial G3 (B) and the control group (C) with 200X magnification (top pictures) and 500X (lower pictures).
**Figure 5** presents representative images of sections stained with hematoxylin and eosin showing the coronal pulp 2 days after bleaching. The panels represent the groups treated with the bleaching gel, the commercial product (G3) for 45 minutes and the control group, with 40x and 100x magnification.

### DETAILED DESCRIPTION OF THE TECHNOLOGY

The present technology relates to a product applied for teeth whitening based on the generation of active oxygen in the form of oxygen radical species in situ. The whitening gel comprises modified niobium compounds and its activity is maximized mainly if it is applied with hydrogen peroxide in low concentrations, from 1 to 10% (preferably 3% by mass), with stabilization by a thickener, preferably carbopol. The whitening gel can be used in teeth whitening without the need for additional light to the treatment, which allows its use in teeth whitening with greater efficiency than commercial products and with a significant potential to reduce side effects (hypersensitivity and inflammation). The developed product did not show cytotoxicity, and no significant inflammatory effect was observed in the pulp tissue. The present technology also describes a method for producing the whitening gel.

The teeth whitening gel comprises niobium compounds modified by previous reaction with peroxides and a thickener, in the ratio between 1 and 50% by mass of the niobium compound in relation to the thickener.

The thickener used can be selected from the group comprising natrosol, xanthan gum, hydroxymethylcellulose, carbomer, carbopol or combinations thereof, or toothpaste or pure glycerin.

The niobium compounds used may be selected from the group comprising niobium phosphates, niobium oxides, acetates, chlorides, niobium filter cake, niobia, or the oxalate anion ([Nb(O)(C₂O₄)_{3]}³⁻).

The peroxides used may be selected from the group comprising methyl ethyl ketone peroxide, benzoyl peroxide, carbamide peroxide, or hydrogen peroxide, with a purity between 30 and 70%, in concentrations between 1, 0 and 10.0% m/m of peroxide in relation to the total mass of the gel (thickener and niobium compound).

The teeth whitening gel preparation process comprises the following steps:
a. Modifying the niobium compounds, generating surface oxidizing oxygen groups by reacting with hydrogen peroxide of purity between 30 and 70%, using peroxide concentrations between 1.0 and 10.0% m/m in relation to the total mass (thickener and compound of niobium);
b. Adding the thickener in the ratio of 1 to 10% by mass of the modified niobium compound obtained in step "a" in relation to the thickener;
c. Mixing the composition obtained in "b" under gentle agitation between 50 and 1000 rpm for a time interval between 10 and 60 min, at room temperature.

In step "a" the niobium compounds are selected from the group comprising niobium phosphates, niobium oxides, acetates, chlorides, niobium filter cake, niobia, or the oxalate anion ([Nb(O)(C₂O₄)_{3]}³⁻).

In step "a" the peroxides are selected from the group comprising methyl ethyl ketone peroxide, benzoyl peroxide, carbamide peroxide or hydrogen peroxide.

In step "b" the thickeners are selected from the group comprising natrosol, xanthan gum, hydroxymethylcellulose, carbomer, carbopol or combinations thereof, or toothpaste or pure glycerin.

The teeth whitening gel can be used to prepare formulations for teeth whitening.

The following examples describe aspects of the present technology and should not be considered as limiting.

### EXAMPLE 1 - Obtaining the whitening gel from the active compound of niobium containing active oxygen.

The whitening gel is obtained by mixing carbopol with the niobium compound, preferably the oxalate, [Nb(O)(C₂O₄)_{3]}³, but it can be niobium pentoxide, niobic acid, niobium phosphate or alternatively niobium oxyhydroxide.

The niobium compounds were modified with commercial hydrogen peroxide (35% V/V) so that in the whitening gel the concentration is 3% by mass of the peroxide in relation to niobium and carbopol. Commercial carbopol was added in order to obtain a gel in the ratio of 1% by mass of the niobium compound in relation to the carbopol. It is important to emphasize that the whitening gel is stabilized by the presence of carbopol, which, in addition to providing the consistency of the gel, stabilizes the oxygenated radicals formed. Reactive species (oxygen radicals) are formed according to the following three chemical reactions:

-Nb-OH + H₂O₂ →-Nb-OOH + H₂O (1)

-Nb-O-O-H→ -Nb-O* + *OH (2)

-Nb-O* + H₂O → -Nb-OH + *OH (3)

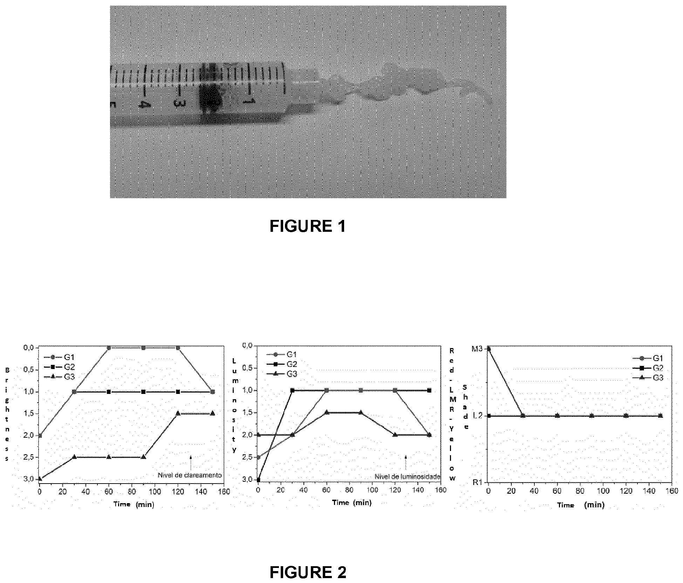

The resulting gel was kept under gentle agitation for 40 min. Figure 1 shows a photograph of the final gel obtained containing the active oxygen species ready for use as a teeth whitener.

### EXAMPLE 2 - Teeth whitening tests in the presence of low H₂O₂ content (2% m/m)

Twelve bovine teeth were selected that were cleaned and preserved in thymol solution (0.1%). The teeth were randomly divided into 6 groups (n=2) and properly identified (Table 1). The initial buccal surface shade for the middle third of each tooth was determined using the VITA Easy Shade spectrophotometer (VITA, Bad Sackingen, Germany).Teeth whitening levels were compared to the VITA 3D Master scale (Vita, Bad Sackingen, Germany). Photographs of bovine teeth were recorded before and after tooth whitening using a Canon T6i camera, macro 100 mm, manual mode, speed 1/160, diaphragm 5.0, ISO 1600, without the use of flash.

**Table 1: Composition of materials applied to teeth whitening**

| **Group** | **Composition** |
|---|---|
| G1 | Carbopol, catalyst (niobium compound), hydrogen peroxide (2%) |
| G2 | Carbopol, catalyst (niobium compound previously treated |
| | with hydrogen peroxide), hydrogen peroxide (2%). |
| G3 | Commercial product - **White Class 6% (H2O2), FGM** |

The bleaching tests were performed using hydrogen peroxide as a bleaching agent and niobium compounds. A commercial product for teeth whitening (White Class 6%, FGM) normally used at home or supervised by the Dental Surgeon was tested as a comparison. For the teeth whitening test, the buccal surface of each specimen was covered by a layer of the mixture containing 4.0 g of Carbopol gel, 1% of the niobium compound and an equivalent amount of 2% of hydrogen peroxide. Applications of the material were carried out at times of 30, 60, 90 and 120 minutes in the absence of light radiation and at the end of each application, the measurement of the color of the teeth was performed with the VITA Easy Shade spectrophotometer. After 120 minutes, the effect of hydration was evaluated in which the teeth were immersed in water for another 30 minutes.

Figure 2 shows the results of teeth whitening that are represented by the parameters of brightness, intensity of color/luminosity and shade obtained through the VITA 3D MASTER scale.

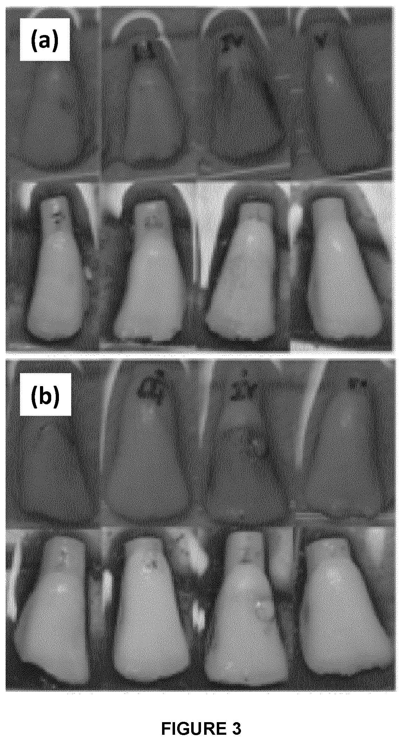

In addition, the effect of teeth whitening in bovine teeth was qualitatively observed using images obtained by a photographic camera. The images that show the effect of applying the materials G1 and G3 on the teeth are shown in Figure 3.

The G1 group was the one that presented the best result compared to the others; a color change was detected already from the evaluations with 30 minutes. One point for the brightness parameter and one point for the luminosity parameter. In the evaluation after 60 minutes of application of the teeth whitening gel, there was a gain of one more point for brightness and two for luminosity. This bleaching level stabilized until the last application, totaling 120 minutes (Figure 2). There was no change for the shade parameter, as expected.

The catalysts with previous chemical treatment (G2) showed lower efficiency in the teeth whitening process when compared to G1. The only recorded evolution of bleaching was only one point in the luminosity parameter. Treatment of the solid with hydrogen peroxide generates reactive oxygen species through the interaction of Bronsted acid sites (Nb-OH) or Nb=O groups, present on the surface of the niobium compound, with H₂O₂. In both materials (G1 and G2) the formation of these species occurs, but the in situ formation of the oxygenated groups, as in G1, potentiates teeth whitening through the efficient oxidation of the dye molecules responsible for the color of the tooth. G3 presented the evolution in whitening in the parameters of brightness and luminosity, 3 and 1 points, respectively. The change in the shade parameter (1 point) was not significant for the assessment of the bleaching level, as can also be seen in Figure 3.

The results of teeth whitening using the teeth whitening gel of the present invention, in the presence of hydrogen peroxide (2%), are similar to bleaching using the commercial product FGM which has a higher concentration of hydrogen peroxide (6%). The gel that presented the best performance for teeth whitening, G1, was tested in the presence of different concentrations of hydrogen peroxide, among them, 0.5 and 1%. Varying the concentration of the bleaching agent in the materials, it was found that the concentration of 2% (G1) maximized the effect of teeth whitening, being more efficient than the commercial material. Lower concentrations of 0.5 and 1% of H₂O₂ showed less expressive results, around 2 points in the bleaching level and were therefore less effective than the commercial product. The reactive oxygen groups formed in situ depend directly on the amount of hydrogen peroxide added to the material, since low concentrations limit the formation of reactive species and consequently the efficiency of teeth whitening is reduced.

The present invention shows that the gel formed with the niobium compound showed a high ability to remove pigmentation in a shorter application time when compared to the commercial product. For comparison purposes, teeth whitening tests were also performed in the absence of the niobium compound and the results showed that the bleaching action was inefficient. Hydrogen peroxide in low concentrations is not enough to whiten teeth and the presence of the niobium catalyst is essential to potentiate the decomposition of hydrogen peroxide forming reactive oxygen species in situ to act in teeth whitening efficiently.

It should be noted that the present invention employs a lower amount of hydrogen peroxide in the teeth whitening process than commercial products widely used by dentists, with significant potential to reduce the side effects of treatment. This fact is of high clinical relevance, since the indiscriminate use of high concentrations of peroxides and prolonged application times can cause undesirable damage to the dental structure, ranging from increased sensitivity to pulp necrosis or degradation of the enamel crystalline structure.

### EXAMPLE 3 - In vivo study of the effect of teeth whitening gel containing niobium on tooth enamel and pulp

In order to evaluate the effect of the teeth whitener on tooth enamel, scanning electron microscopy (SEM) images were obtained. The evaluated teeth were extracted from rats submitted to teeth whitening treatment with teeth whitening gel materials with niobium (G1), commercial (G3) for 45 minutes and with the control group, which consists of teeth that were not submitted to treatment with teeth whitener.

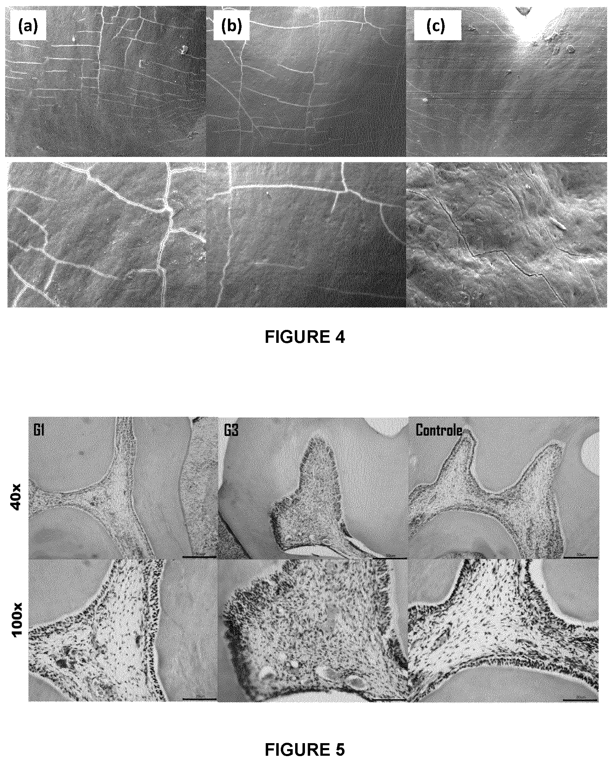

In all the images in Figure 4 it is possible to note the cracks in the enamel of the teeth, including in the control group. This behavior is normal in rat teeth, which are more sensitive than human teeth, and the effect on human teeth is likely to be less pronounced. The EDS spectra show the presence of the elements Ca, O, P, C, Na, Cl, K and Mg in all teeth. The teeth submitted to application with the teeth whitening gel containing niobium (G1) present a Ca/O and Ca/P ratio of 2.9 and 2.3, respectively. These values are higher than those observed for the teeth submitted to commercial dental bleaching (G3), which presented Ca/O=1.5 and Ca/P=2.1 and also what was observed for the control group, in which Ca/O =1.2 and Ca/P=1.95. These results show that the use of niobium-containing teeth whitening gel as a teeth whitener does not harm the chemical composition of the teeth, and consequently does not cause their demineralization. Therefore, it can be said that the material developed does not have an aggressive effect on tooth enamel.

To evaluate changes in the dental pulp, histological examination was performed on teeth of rats after undergoing teeth whitening using the materials G1 (teeth whitening gel containing niobium), G3 (commercial) and the control group. The work procedure with the animals was carried out, respecting the biosafety standards and recommendations of the Ethics Committee of the Federal University of Minas Gerais (CETEA). From the tests performed with the rats of the *Rattus No vergicus* group, the images obtained are shown in Figure 5.

All species of this group exhibited significant changes in the coronal pulp tissue. The images showed a considerable amount of inflammatory cells in the pulp tissue of rats submitted to teeth whitening with the commercial product, containing higher concentration of H₂O₂. In addition, the odontoblast layer is more disorganized for this group, indicating more aggressive effects on the inflammatory process. Inflammation was not significant in rats that were submitted to whitening with the material now developed, exhibiting a similar behavior to the control. In addition, the odontoblastic layer was not altered, showing to be intact, as observed in the control group.

## Claims

1. **A TOOTH-WHITENING GEL characterized by** comprising nanostructured niobium compounds modified by previous reaction with peroxides and a thickener, in the ratio between 1 and 50% by mass of the niobium compound in relation to the thickener.

2. **A TOOTH-WHITENING GEL, according to claim 1, characterized in that** the thickener is selected from the group comprising natrosol, xanthan gum, hydroxymethylcellulose, carbomer, carbopol or combinations thereof, or toothpaste or pure glycerin.

3. **A TOOTH-WHITENING GEL, according to claim 1, characterized in that** the niobium compounds are selected from the group comprising niobium phosphates, niobium oxides, acetates, chlorides, niobium filter cake, niobia, or the oxalate anion ([Nb(O)(C₂O₄)₃]³⁻).

4. **A TOOTH-WHITENING GEL, according to claim 1, characterized in that** the peroxides are selected from the group comprising methyl ethyl ketone peroxide, benzoyl peroxide, carbamide peroxide, or hydrogen peroxide, with a purity between 30 and 70%, in concentrations between 1, 0 and 10.0% m/m of peroxide in relation to the total mass of the gel (thickener and niobium compound).

5. **A METHOD FOR PREPARING THE TOOTH-WHITENING GEL defined in claim 1, characterized by** comprising the following steps:
a) Modifying the niobium compounds with hydrogen peroxide of purity between 70 and 70%, using peroxide concentrations between 1.0 and 10.0% m/m in relation to the total mass (thickener and niobium compound);
b) Adding the thickener in the ratio of 1 to 10% by mass of the modified niobium compound obtained in step "a" in relation to the thickener;
c) Mixing the composition obtained in "b" under gentle agitation between 50 and 1000 rpm for a time interval between 10 and 60 min, at room temperature.

6. **THE METHOD FOR PREPARING THE TOOTH-WHITENING GEL, according to claim 5, step "a", characterized in that** the niobium compounds are selected from the group comprising niobium phosphates, niobium oxides, acetates, chlorides, niobium filter cake, niobia, or the oxalate anion ([Nb(O)(C₂O₄)₃]³⁻).

7. **THE METHOD FOR PREPARING THE TOOTH-WHITENING GEL, according to claim 5, step "a", characterized in that** the peroxides are selected from the group comprising methyl ethyl ketone peroxide, benzoyl peroxide, carbamide peroxide or hydrogen peroxide.

8. **THE METHOD FOR PREPARING THE TOOTH-WHITENING GEL, according to claim 5, step "b", characterized in that** the thickener is selected from the group comprising natrosol, xanthan gum, hydroxymethylcellulose, carbomer, carbopol or combinations thereof, or toothpaste or pure glycerin.

9. **USE OF THE TOOTH-WHITENING GEL defined in claim 1, characterized in that** it is for preparing formulations for teeth whitening.
